(19) **Europäisches Patentamt**
**European Patent Office**
**Office européen des brevets**

(11) **EP 4 755 303 A1**

(12) **EUROPEAN PATENT APPLICATION**

(43) Date of publication:
**10.06.2026   Bulletin 2026/24**

(21) Application number: **24217625.3**

(22) Date of filing: **05.12.2024**

(51) International Patent Classification (IPC):
**A61B 6/00** *(2024.01)*      **A61B 6/03** *(2006.01)*
**A61B 6/42** *(2024.01)*      **A61B 6/50** *(2024.01)*

(52) Cooperative Patent Classification (CPC):
**A61B 6/482; A61B 6/032; A61B 6/4241;**
**A61B 6/504; A61B 6/5217; A61B 6/5258;**
A61B 6/465

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**GE KH MA MD TN**

(71) Applicant: **Siemens Healthineers AG**
**91301 Forchheim (DE)**

(72) Inventors:
• **Wels, Michael**
  **96047 Bamberg (DE)**

• **Jangra, Ashish**
  **124106 Distt. Jhajjar (IN)**
• **Lades, Felix**
  **91052 Erlangen (DE)**
• **Allmendinger, Thomas**
  **91301 Forchheim (DE)**
• **Jürgens, Markus**
  **91325 Adelsdorf (DE)**

(74) Representative: **Siemens Healthineers**
**Patent Attorneys**
**Postfach 22 16 34**
**80506 München (DE)**

(54) **COMPUTER-IMPLEMENTED METHOD FOR PROVIDING IMAGE DATA**

(57)    The invention relates to a computer-implemented method for providing image data based on raw image data of a dual energy computed-tomography arrangement, comprising: providing (10) raw image data by means of a dual energy computed-tomography arrangement comprising low energy image data and high energy image data; providing (11) a tissue classification model, wherein a tissue classification is based on a dual energy ratio of low energy image data and high energy image data; and utilizing (12) the tissue classification model and providing image data, wherein at least the raw image data are input data of the tissue classification model.

## FIG 1

## Description

The present disclosure relates to a computer-implemented method for providing image data based on raw image data of a dual energy computed-tomography arrangement, a system for providing image data based on raw image data of a dual energy computed-tomography arrangement and a corresponding computer program element.

Atherosclerotic changes of the vessel wall may have several manifestations. Depending on the tissue composition these plaques may come with a different risk for a patient. Rupture-prone lesions, for instance, have a lipid-rich core while other compositions indicate erosion-prone or rather stable wall changes. For this reason, it is of interest for patient management to gain insights about the underlying structure of atherosclerotic lesions, ideally in a non-invasive manner. As the general tissue types of interest in this case are calcified, lipid-rich, and/or fibrotic tissue, it would be beneficial to distinguish and quantify these in appropriate medical images.

Computed-tomography angiography has long been drawn into consideration for such a task and in particular dual-energy computed-tomography angiography seems to be promising to provide the required insights. While calcifications may be identified easily with both single-energy and the dual-energy computed-tomography, it is more difficult to reliably separate the soft plaque tissue types from each other. For this purpose, dual- or multi-spectral imaging based on photon counting computed-tomography has been considered superior in comparison to merely dual source-based acquisition as perfect geometric alignment of the different spectral 3D scans is an inherent property of photon counting computed-tomography.

In this context, it has become apparent that there is a further need to provide a method and/or a system providing an improved tissue classification in raw image data of a dual energy computed-tomography arrangement.

It is therefore an object of the present disclosure to provide a method and/or a system providing/allowing an improved tissue classification in raw image data of a dual energy computed-tomography arrangement.

These and other objects, which become apparent upon reading the following description, are solved by the subject matters of the independent claims. The dependent claims refer to preferred embodiments of the present disclosure.

In one aspect of the present disclosure, a computer-implemented method for providing image data based on raw image data of a dual energy computed-tomography arrangement is disclosed, comprising:

> providing raw image data by means of a dual energy computed-tomography arrangement comprising low energy image data and high energy image data;
> providing a tissue classification model, wherein a tissue classification is based on a dual energy ratio of low energy image data and high energy image data; and
> utilizing the tissue classification model and providing image data, wherein at least the raw image data are input data of the tissue classification model.

In other words, the present disclosure proposes to base the plaque tissue classification in the raw image data of a dual energy computed-tomography arrangement on the dual energy ratio of the low energy image data and the high energy image data. In the past, tissue classification was typically based on the dual energy index or on a direct application of HU values (Hounsfield values). The present disclosure is based on the finding that tissue classification based on a dual energy ratio is very suitable to provide reliable tissue classification. Furthermore, a tissue classification based on the dual energy ratio has additional advantages, which are described in the following in the context of the further embodiments.

The term "raw image data" is to be understood broadly in the present case and may include any image data provided by a dual energy computed-tomography arrangement.

The term "dual energy ratio" is also to be understood broadly in the present case and may encompass any ratio of low energy image data and high energy image data.

The term "tissue classification model" is also to be understood broadly in this context and includes simple fall group distinctions or utilization of formulas. However, more complex evaluation models may also be used. In an example, the evaluation model may comprise at least one trained algorithm, wherein the term "trained algorithm" or "trained evaluation model" as used herein is to be understood broadly in the present case. The algorithm may be a machine learning algorithm. The algorithm may comprise decision trees, naive bayes classifications, nearest neighbors, neural networks, convolutional or recurrent neural networks, transformers, generative adversarial networks, support vector machines, linear regression, logistic regression, random forest, gradient boosting algorithms and/or a diffusion model. Such an algorithm, in particular machine learning algorithm, is termed "intelligent" because it is capable of being "trained." The algorithm may be trained using records of training data. A record of training data comprises training input data and corresponding training output data. In the context of the present disclosure, training data may, for example may be sets of raw image data, dual energy ratios and image output data. The training output data of a record of training data is the result that is expected to be produced by the algorithm when being given the training input data of the same record of training data as input. The deviation between this expected result and the actual result produced by the algorithm is observed and rated by means of a "loss function". This loss function may be used as feedback for adjusting the parameters of the internal processing chain of the algorithm. For example, the parameters may be adjusted with the optimization goal of minimizing the values of the loss function that result when all training input data are fed into the algorithm and the outcome is compared with the corresponding training output data. The result of this training is that given a relatively small number of records of training data as "ground truth", the algorithm is enabled to perform its job well for a number of records of input data that is higher by many orders of magnitude. Notably, the evaluation model may comprise several or even a large number of different evaluation routines/sub-models. It is also possible to use mixed evaluation routines that combine different evaluation approaches.

In an embodiment of the computer-implemented method, the low energy image data and the high energy image data may be provided as denoised low energy image data and denoised high energy image data. Noise reduction is the process of removing noise from a signal. There are many noise reduction algorithms in image processing, such as nonlocal means, wavelet transform, iterative reconstruction, Deep-Learning-based approaches and others. Non-local means denoising is a patch-based denoising method that compares patches of similar texture or structure across the image to estimate the noise level in each pixel. Wavelet-based denoising is a popular method for reducing noise in CT images. This method involves decomposing the image into different frequency bands using wavelet transforms and then filtering out the noise in each frequency band before reconstructing the image. Iterative reconstruction (IR) algorithms are the most widely used CT noise-reduction method to improve image quality. There are several Deep-Learning approaches for CT image denoising, including but not limited to: convolutional neural networks (CNNs), Generative Adversarial Networks (GANs), Variational Autoencoders (VAEs), Deep Residual Networks (ResNets), Transformer-based methods, Attention-based Networks, as well as hybrid approaches that combine multiple approaches, such as CNNs and GANs, to improve the quality and accuracy of CT image denoising.

In an embodiment of the computer-implemented method, the dual energy ratio may be calculated according to the following formula:

$$r = \frac{x_L - u_L}{x_H - u_H};$$

with

$x_L$ :  CT value from low energy image data, preferably from the denoised low energy image data;

$x_H$:  CT value from high energy image data, preferably from the denoised high energy image data;

$u_L$:  predefined CT value for fatty regions for the low energy image data; and

$u_H$:  predefined CT value for fatty regions for the high energy image data.

In an embodiment of the computer-implemented method, for CT values from high energy image data $x_H$ which are greater than a predetermined buffer value $u_{HB}$, the dual energy ratio r may be set to 0, wherein the buffer value $u_{HB}$ is preferably set between -89 HU and -95 HU, more preferably between -91 HU and -93 HU and most preferably at -92 HU. Thereby, the influence of noise for $x_H$ values close to -92 HU may be reduced.

In an embodiment of the computer-implemented method, $u_L$ may be set between -95 HU and -105 HU, preferably between -98 HU and -102 HU and most preferably at -100 HU.

In an embodiment of the computer-implemented method, $u_H$ may be set between -87 HU and -97 HU, preferably between -90 HU and 94 HU and most preferably at -92 HU.

In an embodiment of the computer-implemented method, the dual energy ratio r may be assigned as follows:

dual energy ratios r ≥ 1.42 amounts to pure calcium;
dual energy ratios r ≤ 1.0 is fat; and
dual energy ratios in-between 1.0 and 1.42 indicate some mixture tissue.

In an embodiment of the computer-implemented method, a dual energy threshold r* may be provided according to the following formula:

$$r^* = \frac{\delta_L^*}{\delta_H^*}$$

and $u_{H^B}^*$ by

a position of a point $p^*_{H,L}$ for changing $u_L^*$ and $u_H^* = u_{H^B}^*$ and

a rotation $\alpha$ around the point $p^*_{H,L}$ , with $\delta_H^* > 0$ and $\delta_L^* \geq 0$ .

In an embodiment of the computer-implemented method, a first tissue type may be defined by the region r ≥ r* and a second tissue type may be defined by the region r < r*.

In an embodiment of the computer-implemented method, the predefined CT value for fatty regions for the low energy image data $u_L$, the predefined CT value for fatty regions for the high energy image data $u_H$, the dual energy ratios assigned to different tissues, the dual energy threshold r*, the predetermined buffer value $u_{HB}$, the threshold buffer value $u_{HB}$, the position of the point $p^*_{H,L}$, the rotation $\alpha$, delta $\delta_L^*$ and/or the delta $\delta_H^*$ may be provided by means of a user interface. In this example, a user may, e.g. by means of an interactive graphical user interface, a widget, further analyze suspicious vessel wall regions with regards to their soft plaque composition. Such an interactive graphical user interface may be provided as an editor to configure dual energy ratio-based tissue separation in a case-specific manner. This way a user may interactively and intuitively consider acquisition-dependent, e.g. scan parameters, contrast agent concentration, etc., characteristics yielding understandable tissue classifications and associated volumetric measurements from the dual energy computed-tomography arrangement

Such an interactive component may provide immediate intuition of the current configuration and even the less experienced clinical scientist may be able to find configurations that sufficiently well separate soft plaque tissue components in the scans at hand. These scans may have site-specific characteristics due to differing acquisition where tissue separation would be difficult otherwise. Additionally, biological variation from blooming and beam hardening of the contrasted vessel or nearby calcium may ask for very specific dual energy ratio-based thresholding. In this example, initial and automatically suggested case-specific pre-configurations may be conceivable, which could then be adapted manually by means of a user interface. In contrast to black box approaches, the present disclosure may provide a maximal transparency, i.e. the "decision" to assign sub-voxels to one or the other tissue type may be easy understandable.

In an embodiment of the computer-implemented method, the predefined CT value for fatty regions for the low energy image data $u_L$, the predefined CT value for fatty regions for the high energy image data $u_H$, the dual energy ratios assigned to different tissues, the dual energy threshold r*, the predetermined buffer value $u_{HB}$, the threshold buffer value $u_{HB}$, the position of the point $p^*_{H,L}$, the rotation $\alpha$, delta $\delta^*_L$ and/or the delta $\delta^*_H$ may be provided as predetermined parameters. As already mentioned, these parameters may be initially and automatically provided as case-specific pre-configurations, which may then be adapted manually.

Such interactive and transparency aspect of the present disclosure may become important when it comes to achieving tissue separation exactly according to the users' expectation, which in general supports the acceptance of such analysis tools. In certain cases, it may even be a regulatorily required feature to allow manual overriding of results generated by an automatic preprocessing method of any kind, e.g. model fitting, artificial intelligence, etc.

In an embodiment of the computer-implemented method, the predefined CT value for fatty regions for the low energy image data $u_L$, the predefined CT value for fatty regions for the high energy image data $u_H$, the dual energy ratios assigned to different tissues, the dual energy threshold r*, the predetermined buffer value $u_{HB}$, the

threshold buffer value $u_{HB}$, the position of the point $p^*_{H,L}$, the rotation $\alpha$, delta $\delta^*_L$ and/or the delta $\delta^*_H$ may be provided by a trained algorithm. Such a trained algorithm may be trained, for example, for a specific application or a specific dual energy computed-tomography arrangement.

Low energy image data and high energy image data may be obtained by any dual energy or multi-energy imaging method, such as photon-counting detection, dual-source CT; rapid kV-switching CT; dual-layer CT; sequential acquisitions using different X-ray tube voltages; split-filter CT.

Photon-counting detection allows spectrally resolved capture of raw X-ray data. In dual-source CT, image acquisitions of one and the same region under examination are performed using at least two different average X-ray energies. Rapid kV-switching CT involves switching the X-ray source between different X-ray energies, so that a plurality of image acquisitions can be performed using different X-ray energies. In dual-layer CT, the detector consists of two or more layers, each of which detects just part of the X-ray spectrum. In sequential acquisitions using a different voltage, the same acquisition is performed twice in quick succession, with the X-ray energy being switched between the acquisitions. In split-filter CT, two different filters, for instance gold and tin, split the X-ray beam longitudinally into two regions, one with a lower spectrum and one with a higher spectrum. Thus in the case of multirow CT, half of the rows detect the low-spectrum signal and the other half detect the high-spectrum signal.

In an embodiment of the computer-implemented method, dual energy computed-tomography arrangement is a photon counting computed-tomography arrangement. The present disclosure may leverage the value of photon counting for quantitative high-risk plaque assessment from angiographic scans in a non-invasive manner.

A further aspect of the present disclosure relates to a system for providing image data based on raw image data of a dual energy computed-tomography arrangement comprising:

   a processing circuitry;
   a storage medium; and
   a data interface;
   wherein the storage medium comprises a computer program that comprises instructions which when the program is executed, cause the processing circuitry to carry out the above-explained computer-implemented method;
   wherein the data interface is configured to receive input data for the tissue classification model and the raw image data.

A further aspect of the present disclosure relates to a computer program element with instructions, which, when executed on computing devices of a computing environment, is configured to carry out the steps of the above-described computer-implemented in an above-described system. As mentioned, such a computer program element may be provided as interactive graphical user interface, e.g. a widget element. The computer program element might be stored on a computing unit of a computing device, which might also be part of an embodiment. This computing unit may be configured to perform or induce performing of the steps of the method described above. Moreover, it may be configured to operate the components of the above-described system. The computing unit can be configured to operate automatically and/or to execute the orders of a user. The computing unit may include a data processor. A computer program may be loaded into a working memory of a data processor. The data processor may thus be equipped to carry out the method according to one of the preceding embodiments. This exemplary embodiment of the present disclosure covers both, a computer program that right from the beginning uses the present disclosure and computer program that by means of an update turns an existing program into a program that uses the present disclosure. Moreover, the computer program element might be able to provide all necessary steps to fulfill the procedure of an exemplary embodiment of the method as described above. According to a further exemplary embodiment of the present disclosure, a computer readable medium, such as a CD-ROM, USB stick, a downloadable executable or the like, is presented wherein the computer readable medium has a computer program element stored on it which computer program element is described by the preceding section. A computer program may be stored and/or distributed on a suitable medium, such as an optical storage medium or a solid-state medium supplied together with or as part of other hardware, but may also be distributed in other forms, such as via the internet or other wired or wireless telecommunication systems. However, the computer program may also be presented over a network like the World Wide Web and can be downloaded into the working memory of a data processor from such a network. According to a further exemplary embodiment of the present disclosure, a medium for making a computer program element available for downloading is provided, which computer program element is arranged to perform a method according to one of the previously described embodiments of the present disclosure.

This and embodiments described herein relate to the methods, the systems, the apparatuses, the computer program element, the computer-readable storage medium, the use lined out above and vice versa. Advantageously, the benefits provided by any of the embodiments and examples equally apply to all other embodiments and examples and vice versa. In other words, claims for the system according to the invention can be improved with features described or claimed in the context of the methods and vice versa. In this case, the functional features of the method are embodied by objective units or modules of the system.

The term "data" as used herein is to be understood broadly in the present case and represents any kind of data. Data may be single numbers/numerical values, a plurality of a numbers/numerical values, a plurality of a numbers/numerical values being arranged within a list, 2 dimensional maps or 3 dimensional maps, but are not limited thereto.

The term "providing" as used herein is to be understood broadly in the present case and represents any providing, receiving, querying, measuring, calculating, determining, transmitting of data, but is not limited thereto. Data may be provided by a user via a user interface, depicted/shown to a user by a display, and/or received from other devices, queried from other devices, measured other devices, calculated by other device, determined by other devices and/or transmitted by other devices.

In the following particularly preferred embodiments are disclosed, which may be combined with the above-disclosed methods, systems, apparatuses, devices and/or use cases. The embodiments described herein may be combined unless specifically noted otherwise. Features and advantages described with respect to one aspect of the disclosure may be applied to other aspects of the disclosure.

In the following, the present disclosure is further described with reference to the enclosed figures:

**Figure 1** illustrates a flow diagram of a computer-implemented method for providing image data based on raw image data of a dual energy computed-tomography arrangement according to an embodiment of the present disclosure;

**Figure 2** illustrates a system for providing image data based on raw image data of a dual energy computed-tomography arrangement according to an embodiment of the present disclosure;

**Figure 3** illustrates an interactive user interface according to an embodiment of the present disclosure;

**Figure 4** illustrates a geometry of a vessel cross-section with the tissue types lipid-rich, fibrotic, and vessel lumen;

**Figure** 5 illustrates a low kV image from the vessel cross-section shown in Figure 4;

**Figure 6** illustrates a high kV image from the vessel cross-section shown in Figure 4; and

**Figure 7** illustrates a 2D histogram.

The following embodiments are mere examples for implementing the method, the system, disclosed herein and shall not be considered limiting.

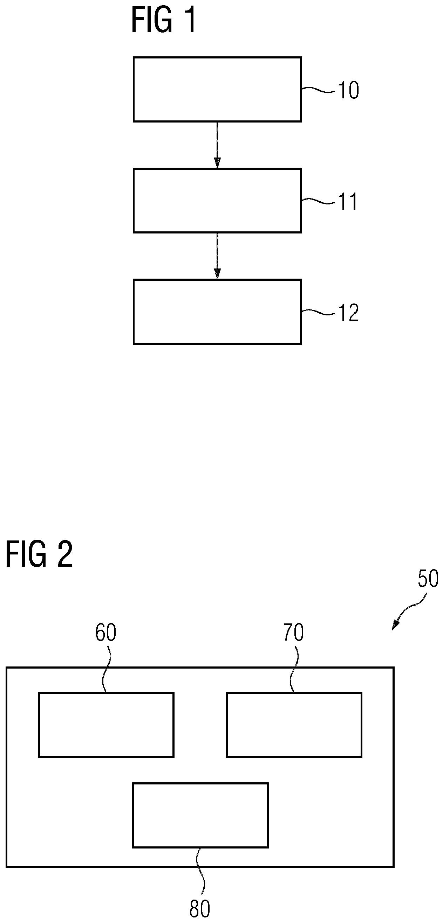

**Figure 1** illustrates a flow diagram of a computer-implemented method for providing image data based on raw image data of a dual energy computed-tomography arrangement according to an embodiment of the present disclosure according to an embodiment of the present disclosure. In a first step 10, raw image data provided by means of a dual energy computed-tomography arrangement comprising low energy image data and high energy image data are provided. In a second step 11, a tissue classification model is provided, wherein a tissue classification is based on a dual energy ratio of low energy image data and high energy image data. In a further step 12, the tissue classification model is utilized and image data are provided, wherein at least the raw image data are input data of the tissue classification model.

**Figure 2** illustrates a system 50 for providing image data based on raw image data of a dual energy computed-tomography arrangement according to an embodiment of the present disclosure, comprising: a processing circuitry 60, a storage medium 70, and a data interface 80. The storage medium 70 comprises a computer program with instructions which when the program is executed, cause the processing circuitry 60 to carry out the computer-implemented method for providing image data based on raw image data of a dual energy computed-tomography arrangement according to an embodiment of the present disclosure according to an embodiment of the present disclosure. The data interface 80 is configured to receive at least input data for the tissue classification model and the raw image data

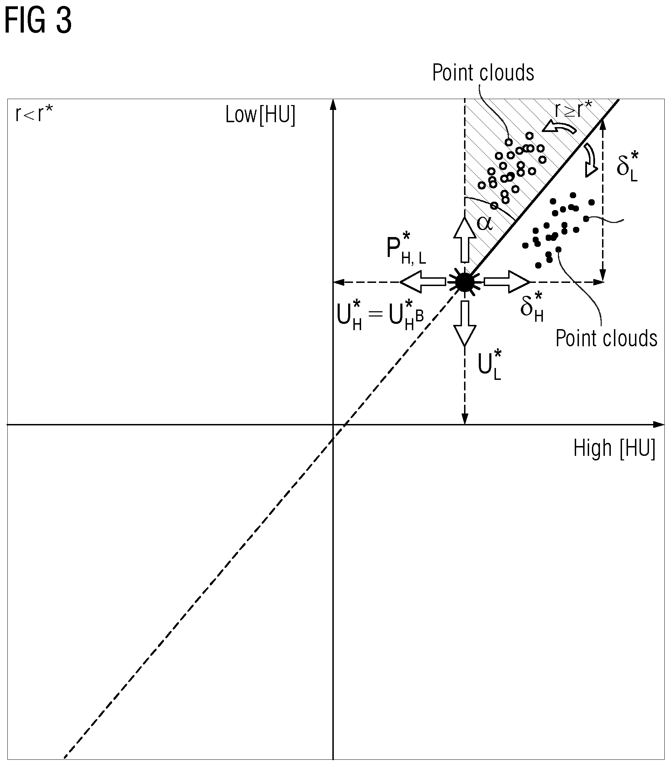

**Figure 3** is a schematically illustration of an interactive user interface. The user interface comprises a 2D histogram where the potentially subsampled voxels of a volume of interest are plotted as point clouds depending on their HU values in the denoised high kV image and low kV image, wherein an interactive user interface element is provided comprising a transferrable point $p^*_{H,L}$ from which a rotatable line originates to the upper-right. The degree of rotation is quantified by the angle $\alpha$ relative to the vertical axis. The user interface can be used to partition the feature space into the shown two regions. In the shown example, the dual energy ratio threshold

$$r^* = \frac{\delta^*_L}{\delta^*_H}$$

and $u^*_{H^B}$ by

- the position of the point $p^*_{H,L}$ for changing $u^*_L$ and $u^*_H = u^*_{H^B}$ and

- the rotation $\alpha$ around $p^*_{H,L}$ (with $\delta^*_H > 0$ and $\delta^*_L \geq 0$.

This yields two regions $r \geq r^*$ and $r < r^*$ separating two tissue types. This separation may be also used to classify and to quantify plaque compartments in 3D. Also, additional separation lines not necessarily anchored at $p^*_{H,L}$ for, e.g., initial calcium separation and other tissue types are conceivable. A possible generalization, may be to define a cascade of dual energy ratio thresholding operations through multiple interactive user interface elements and associated $r^*_i$ on top of each other on the same diagram. Moreover, even further higher-level visualizations could be interactively configured: For instance, a color heatmap overlay or segmentation may become conceivable, which is based on the calcification index c = 100% * (r- $r_{fat}$)/($r_{ca}$ - $r_{fat}$), where the configuration of the first interactive user element may be used for the computation of r and optional additional two elements for $r_{ca}$ and $r_{fat}$ in case they need to be interactively adaptable.

In the shown example, at least the position of the point $p^*_{H,L}$ and the rotation $\alpha$ around $p^*_{H,L}$ may be changed by a user. Moreover, in the shown example, the dual energy ratio on which a tissue type classification is based, is described as follows:

The dual energy ratio be $r = \frac{x_L - u_L}{x_H - u_H}$ for $x_H > u_{HB}$

*and* r = 0 otherwise with

- $x_L$, $x_H$: CT values from L0 and H0
- $u_L$, $u_H$: CT values for fatty regions for the low and the high energy image (E.g., choose $u_L$ = -100 HU, $u_H$ = -92 HU as initial settings for P63A 120kV scans.)
- $u_{HB}$: buffer value to reduce the influence of noise for $x_H$ values close to -92 HU

In the shown example, r $\geq$ 1.42 amounts to pure calcium, $r \leq$ 1.0 is fat, and ratios in-between indicate some mixture.

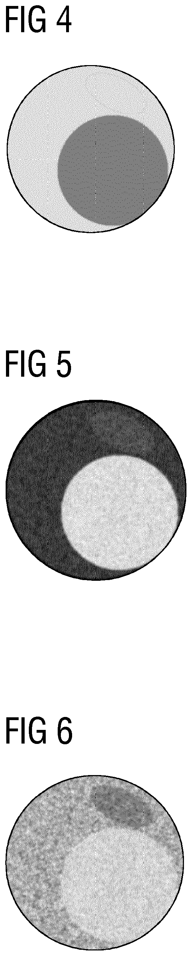

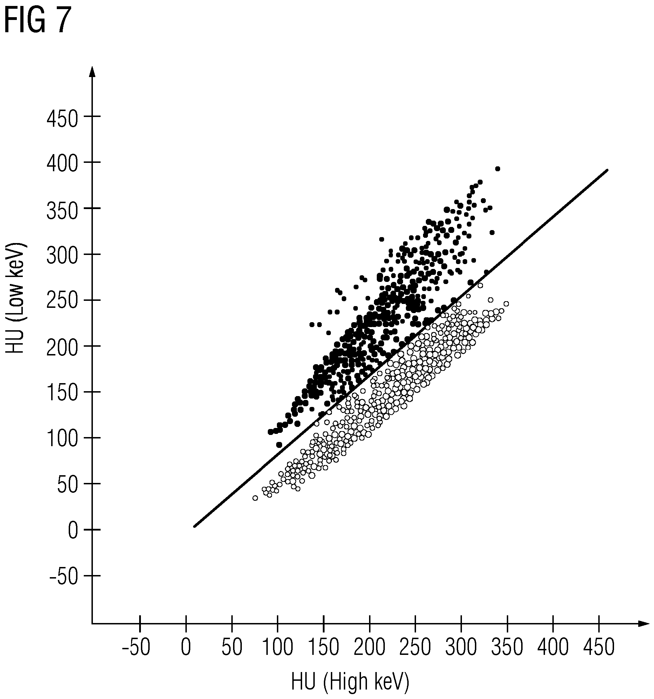

**Figure 4** illustrates a geometry of a vessel cross-section with the tissue types lipid-rich, fibrotic, and vessel lumen. **Figure 5** illustrates the low kV image and **Figure 6 the** high kV image from the vessel cross-section shown in **Figure 4** with assumed, ideal-typical HU values. In **Figure 7** the associated 2D histogram is illustrated, wherein the tissue types based on the above explained dual energy ratio and the dual energy ratio threshold are illustrated in **Figure 7**. As shown, the by means of the interactive user interface an almost perfectly separation of the two soft tissue types may be provided.

The present disclosure has been described in conjunction with a preferred embodiment as examples as well. However, other variations can be understood and effected by those persons skilled in the art and practicing the claimed invention, from the studies of the drawings, this disclosure and the claims. Notably, in particular, the any steps presented can be performed in any order, i.e. the present invention is not limited to a specific order of these steps. Moreover, it is also not required that the different steps are performed at a certain place or at one node of a distributed system, i.e. each of the steps may be performed at a different node using different equipment/-data processing units.

As used herein "determining" also includes "estimating, calculating, initiating or causing to determine", "generating" also includes "initiating or causing to generate" and "providing" also includes "initiating or causing to determine, generate, select, send, query or receive".

In the claims as well as in the description the word "comprising" does not exclude other elements or steps and the indefinite article "a" or "an" does not exclude a plurality. A single element or other unit may fulfill the functions of several entities or items recited in the claims. The mere fact that certain measures are recited in the mutual different dependent claims does not indicate that a combination of these measures cannot be used in an advantageous implementation. Furthermore, it should be noted that, regardless of the grammatical gender of a particular term, it includes persons with male, female or other gender identities.

**Claims**

1. Computer-implemented method for providing image data based on raw image data of a dual energy computed-tomography arrangement, comprising:

   providing (10) raw image data by means of a dual energy computed-tomography arrangement comprising low energy image data and high energy image data;
   providing (11) a tissue classification model, wherein a tissue classification is based on a dual energy ratio of low energy image data and high energy image data; and
   utilizing (12) the tissue classification model and providing image data, wherein at least the raw image data are input data of the tissue classification model.

2. Computer-implemented method according to claim 1, wherein the low energy image data and the high energy image data are provided as denoised low energy image data and denoised high energy image data.

3. Computer-implemented method according to claim 1 or claim 2, wherein the dual energy ratio is calculated according to the following formula:

$$r = \frac{x_L - u_L}{x_H - u_H};$$

with

$x_L$ : CT value from low energy image data, preferably from the denoised low energy image data;

$x_H$: CT value from high energy image data, preferably from the denoised high energy image data;

$u_L$: predefined CT value for fatty regions for the low energy image data; and

$u_H$ : predefined CT value for fatty regions for the high energy image data.

4. Computer-implemented method according to claim 3, wherein for CT values from high energy image data $x_H$ which are greater than a predetermined buffer value $u_{HB}$, the dual energy ratio r is set to 0, wherein the buffer value $u_{HB}$ is preferably set between -89 HU and -95 HU, more preferably between -91 HU and -93 HU and most preferably at -92 HU.

5. Computer-implemented method according to claim 3 or claim 4, wherein $u_L$ is set between -95 HU and -105 HU, preferably between -98 HU and -102 HU and most preferably at - 100 HU.

6. Computer-implemented method according to any one of claims 3 to 5, wherein $U_H$ is set between -87 HU and -97 HU, preferably between -90 HU and 94 HU and most preferably at -92 HU.

7. Computer-implemented method according to any one of claims 3 to 6, wherein the dual energy ratio r is assigned as follows:

   dual energy ratios r ≥ 1.42 amounts to pure calcium;
   dual energy ratios r ≤ 1.0 is fat; and
   dual energy ratios in-between 1.0 and 1.42 indicate some mixture tissue.

8. Computer-implemented method according to any one of claims 3 to 6, wherein a dual energy threshold r* is provided according to the following formula:

$$r^* = \frac{\delta_L^*}{\delta_H^*}$$

and $u_{HB}^*$ by

a position of a point $p^*_{H,L}$ for changing $u_L^*$ and $u_H^* = u_{HB}^*$ and

a rotation $\alpha$ around the point $p^*_{H,L}$, with $\delta^*_H > 0$ and $\delta^*_L \geq 0$.

9. Computer-implemented method according to claim 8, wherein a first tissue type is defined by the region r $\geq$ r* and a second tissue type is defined by the region r < r*.

10. Computer-implemented method according to any one of claims 3 to 9, wherein the predefined CT value for fatty regions for the low energy image data $u_L$, the predefined CT value for fatty regions for the high energy image data $u_H$, the dual energy ratios assigned to different tissues, the dual energy threshold r*, the predetermined buffer value $u_{HB}$, the threshold buffer value $u_{HB}$, the position of the point $p^*_{H,L}$, the rotation $\alpha$, delta $\delta^*_L$ and/or the delta $\delta^*_H$ are provided by means of a user interface.

11. Computer-implemented method according to any one of claims 3 to 10, wherein the predefined CT value for fatty regions for the low energy image data $u_L$, the predefined CT value for fatty regions for the high energy image data $u_H$, the dual energy ratios assigned to different tissues, the dual energy threshold r*, the predetermined buffer value $u_{HB}$, the threshold buffer value $u_{HB}$, the position of the point $p^*_{H,L}$, the rotation $\alpha$, delta $\delta^*_L$ and/or the delta $\delta^*_H$ are provided as predetermined parameters.

12. Computer-implemented method according to any one of claims 3 to 11, wherein the predefined CT value for fatty regions for the low energy image data $u_L$, the predefined CT value for fatty regions for the high energy image data $u_H$, the dual energy ratios assigned to different tissues, the dual energy threshold r*, the predetermined buffer value $u_{HB}$, the threshold buffer value $u_{HB}$, the position of the point $p^*_{H,L}$, the rotation $\alpha$, delta $\delta^*_L$ and/or the delta $\delta^*_H$ are provided by a trained algorithm.

13. Computer-implemented method according to any one of the preceding claims, wherein dual energy computed-tomography arrangement is a photon counting computed-tomography arrangement.

14. System (50) for providing image data based on raw image data of a dual energy computed-tomography arrangement, comprising:

   a processing circuitry (60);
   a storage medium (70); and
   a data interface (80);
   wherein the storage medium (70) comprises a computer program that comprises instructions which when the program is executed, cause the processing circuitry (60) to carry out the method according to any one of claims 1 to 13; wherein the data interface (80) is configured to receive input data for the tissue classification model and the raw image data.

15. Computer program element with instructions, which, when executed on computing devices of a computing environment, is configured to carry out the steps of the computer-implemented method according to any one of the claims 1 to 13 in a system according to claims 14.

# FIG 1

# FIG 2

# FIG 3

Point clouds

$r < r^*$        Low[HU]

$r \geq r^*$

$\delta_L^*$

$\alpha$

$P_{H,L}^*$

$U_H^* = U_{H^B}^*$

$\delta_H^*$

$U_L^*$

Point clouds

High [HU]

FIG 4

FIG 5

FIG 6

FIG 7

Europäisches
Patentamt

European
Patent Office

Office européen
des brevets

## EUROPEAN SEARCH REPORT

**Application Number**

EP 24 21 7625

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 3 420 902 B1 (SIEMENS HEALTHCARE GMBH [DE]) 23 August 2023 (2023-08-23) | 1,3-7, 10-12, 14,15 | INV.<br>A61B6/00<br>A61B6/03 |
| Y | * abstract * | 2,13 | A61B6/42 |
| A | * paragraph [0001] *<br>* paragraph [0016] *<br>* paragraph [0022] *<br>* paragraph [0031] *<br>* paragraph [0033] *<br>* paragraph [0047] - paragraph [0048] *<br>* paragraph [0052] *<br>* figures 4-6 * | 8,9 | A61B6/50 |
| Y | CYNTHIA H. MCCOLLOUGH ET AL: "Dual- and Multi-Energy CT: Principles, Technical Approaches, and Clinical Applications", RADIOLOGY, vol. 276, no. 3, 1 September 2015 (2015-09-01), pages 637-653, XP055452767, US ISSN: 0033-8419, DOI: 10.1148/radiol.2015142631 | 2,13 | |
| | | | **TECHNICAL FIELDS SEARCHED (IPC)**<br><br>A61B |
| A | * abstract *<br>* section "Energy Domain Noise Reduction" * | 1,3-12, 14,15 | |
| A | US 2018/165811 A1 (FLOHR THOMAS [DE] ET AL) 14 June 2018 (2018-06-14)<br>* paragraph [0002] *<br>* paragraph [0075] - paragraph [0077] *<br>* paragraph [0084] *<br>* abstract * | 1-15 | |

-/--

The present search report has been drawn up for all claims

| Place of search | Date of completion of the search | Examiner |
|---|---|---|
| Munich | 11 April 2025 | Montes, Pau |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 24 21 7625

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | LAMBERT JACK W. ET AL: "The Effect of Patient Diameter on the Dual-Energy Ratio of Selected Contrast-Producing Elements", JOURNAL OF COMPUTER ASSISTED TOMOGRAPHY, vol. 41, no. 3, 1 May 2017 (2017-05-01), pages 505-510, XP093269461, US ISSN: 0363-8715, DOI: 10.1097/RCT.0000000000000557 * abstract * * section "Materials and methods" * ----- | 1-15 | |

TECHNICAL FIELDS
SEARCHED        (IPC)

The present search report has been drawn up for all claims

| Place of search | Date of completion of the search | Examiner |
|---|---|---|
| Munich | 11 April 2025 | Montes, Pau |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 24 21 7625

This annex lists the patent family members relating to the patent documents cited in the above-mentioned European search report.
The members are as contained in the European Patent Office EDP file on
The European Patent Office is in no way liable for these particulars which are merely given for the purpose of information.

11-04-2025

| Patent document cited in search report | | Publication date | Patent family member(s) | | Publication date |
|---|---|---|---|---|---|
| EP 3420902 | B1 | 23-08-2023 | NONE | | |
| US 2018165811 | A1 | 14-06-2018 | CN | 108209955 A | 29-06-2018 |
| | | | EP | 3332710 A1 | 13-06-2018 |
| | | | US | 2018165811 A1 | 14-06-2018 |

EPO FORM P0459

For more details about this annex : see Official Journal of the European Patent Office, No. 12/82